Europäisches Patentamt

**European Patent Office**

**Office européen des brevets**

(19)

(11) Publication number: **0 027 154
B1**

(12) **EUROPEAN PATENT SPECIFICATION**

(45) Date of publication of patent specification: **29.06.83**

(51) Int. Cl.³: **A 63 B 23/00, A 61 B 5/08**

(21) Application number: **79302173.4**

(22) Date of filing: **10.10.79**

(54) Volumetric respiratory exerciser and kit for assembling such an exerciser.

(43) Date of publication of application:
**22.04.81 Bulletin 81/16**

(45) Publication of the grant of the patent:
**29.06.83 Bulletin 83/26**

(84) Designated Contracting States:
**AT BE CH DE FR GB IT LU NL SE**

(56) References cited:
**DD - A - 59 611
DE - C - 459 991
FR - A - 908 444
US - A - 3 859 190
US - A - 3 669 097
US - A - 3 848 583**

(73) Proprietor: **Becton, Dickinson and Company
Mack Centre Drive
Paramus New Jersey 07652 (US)**

(72) Inventor: **Verkaart, Wesley Howard
4 Warren Street Lincoln Park
New Jersey (US)**

(74) Representative: **Ruffles, Graham Keith et al,
MARKS & CLERK 57-60 Lincoln's Inn Fields
London WC2A 3LS (GB)**

Courier Press, Leamington Spa, England

Volumetric respiratory exerciser and kit for assembling such an exerciser

The present invention relates to pulmonary equipment, and in particular relates to volumetric respiratory exercisers.

In U.S.—A—3589190, there is described a respiration testing apparatus suitable for use in pulmonary function analysis. The apparatus includes the outer casing and a horizontal bellows which has a fixed end plate secured to the casing and a movable end plate supported by an elongated, horizontally extending rod which passes through the centre of the movable end plate and is secured thereto. A breathing tube extends through the casing and the stationary end plate into the bellows. The support rod is slidably supported for horizontal movement by a pair of rollers, one of which is rotatably mounted on the casing adjacent one end of the rod and the other end of the rod. The first end of the rod carries a stylus which describes a recording chart to record movement of the rod and the bellows as air is drawn into and removed from the bellows through the breathing tube.

In U.S.—A—3669097 there is described a device for increasing the capacity and strength of lungs. The device has an expansible bellows chamber, an inlet to the chamber connected to a conduit, a mouthpiece at the end of the conduit for breathing into it, and a selectively adjustable valve in the conduit for constricting the passage from the mouthpiece to the inlet, whereby a force in excess of the normal pressure developed by the lungs is required to expand the bellows, and an outlet for said chamber with a valve and variable control therefor adapted to open the outlet when the bellows has been expanded a preselected amount.

In accordance with the present invention, there is provided a volumetric inspiration respiratory exerciser comprising a bellows enclosure with opposed end plates and defining a closed chamber of variable volume, the bellows enclosure being expandable and retractable between expanded and retracted configurations and the bellows enclosure assuming its expanded configuration when the exerciser is ready for use; a conduit in communication at one end with the chamber and having at its other end a mouth piece assembly comprising an air inlet for controlled admission of air into the chamber and a mouth piece by means of which air is withdrawn from the chamber upon inspiration of the user; a support frame for the bellows enclosure which frame allows movement of the bellows enclosure between its expanded and retracted configurations while retaining in a fixed position one of the end plates of the bellows enclosure, such that the bellows enclosure is suspended from the support frame to assume its expanded configuration; and indicia disposed on the frame for indicating the volume of the chamber as it varies with movement relative to the fixed end of the bellows enclosure during inspiration by the user.

Such an exerciser is useful as an exerciser for the lung and respiratory system of humans, and permits monitoring of each inspiration and direct measurement of each inspired volume. Furthermore, the exerciser may be made as a unitary portable apparatus intended for use by an individual patient and can be reasonably compact for transportation, storage, use and disposal.

When the exerciser is set up and ready for use, the bellows enclosure assumes its expanded configuration. Upon evacuation of air from within the chamber by closure of the air inlet and inspiration through the mouth piece, the bellows enclosure is moved towards its retractive configuration. At the end of the inspiration, the indicia can be read to provide the inspired volume.

The exerciser is designed such that the bellows enclosure is suspended from the support frame and thereby assumes its expanded configuration. It is particularly convenient if the bellows enclosure is suspended at or adjacent the said one of the end plates which is to be retained in a fixed position by the support frame.

The bellows enclosure is suitably constructed from flexible materials such as synthetic polymers, and preferably the exerciser is designed so that the other end plate of the bellows enclosure moves in a parallel manner away from and towards the fixed end plate.

In the mouth piece assembly it is preferred to employ a branched conduit such as a "T"-piece, with one opening of the branched conduit forming the air inlet, a second opening being connected to the mouth piece itself, and the third opening being joined to the conduit in communication with the chamber. By appropriate control of the air inlet, the user of the exerciser can control the way in which he expends effort against the bellows. Thus the air inlet is blocked during each inspiration. Once the inspiration volume has been measured, the air inlet can be unclosed so as to allow entry of fresh air and thus allow movement of the bellows enclosure back to its expanded configuration.

Where the exerciser is intended to be used only for the measurement of inspiration volume, it is preferred to include in the mouth piece assembly a one way valve which permits only the inspiration.

The conduit in communication with the chamber is preferably in the form of a flexible hose. It is also preferred that the conduit communicates with the chamber through the fixed end plate of the bellows enclosure.

It is particularly advantageous if the support frame is designed for use as a container for packaging of the other components during transportation of the exerciser.

To this end, the present invention further provides a kit for use in assembling an exerciser in accordance with the invention. The kit comprises a container containing the bellows enclosure and conduit with mouth piece assembly, as required for constructing an exerciser of the invention, with the container being adaptable for use as the required support frame to complete the exerciser.

It is a simple matter for the frame to be used to enclose the bellows enclosure and conduit with mouth piece assembly; the exerciser can then be unpacked and assembled when required for use.

In a preferred embodiment the support frame comprises a carton and a base component.

The support frame can have a notch for mating with a suitable member on the bellows enclosure. To this end, the mating member can take the form of a flange associated with the said one end plate so as to provide a neck of reduced cross-section between the flange and plate and which will fit in the notch.

An exerciser in accordance with the present invention will now be described by way of nonlimiting example with reference to the accompanying drawings in which:

Figure 1 is an isometric view of a preferred embodiment of the invention, the embodiment comprising a bellows enclosure and conduit with mouth piece which are packaged within a container intended for use as a support frame;

Figure 2 shows the bellows enclosure and conduit with mouth piece when removed from their container and ready for assembly with the support frame made from the container components,

Figure 3 shows the assembled exerciser of Figures 1 and 2;

Figure 4 shows a side elevation, partly in cross-section, of the exerciser of Figure 3 during use;

Figure 5 is a view as seen in Figure 4 but with the exerciser in its relaxed configuration;

Figure 6 is a cross-section of the mouth piece assembly of the exerciser of the previous Figures; and

Figure 7 shows an alternative way of packaging the components in the container.

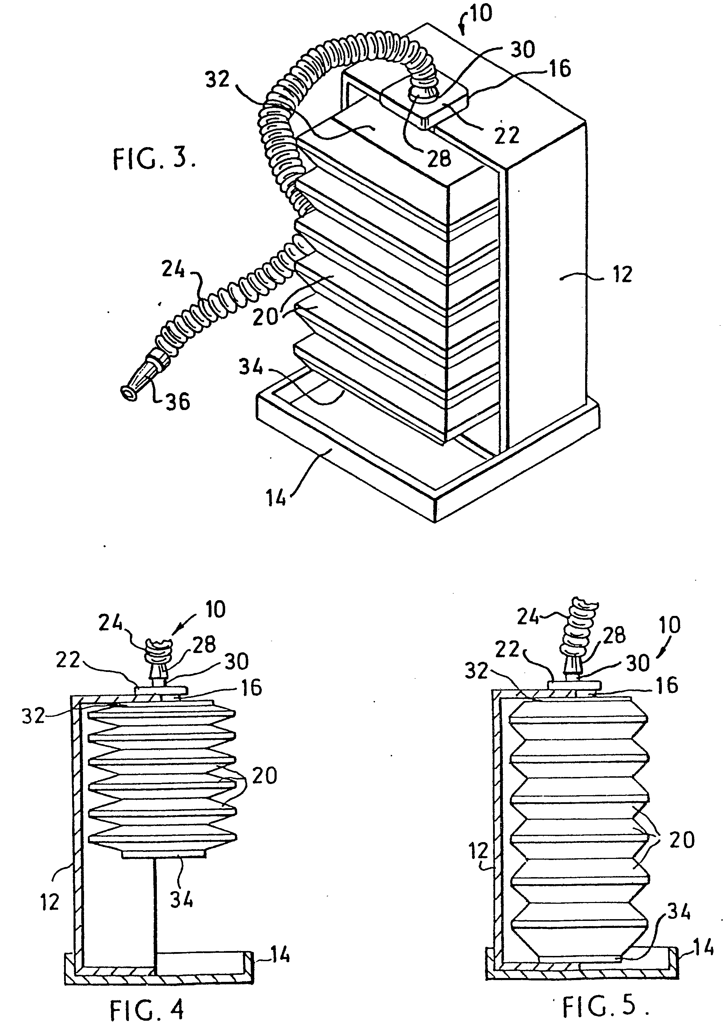

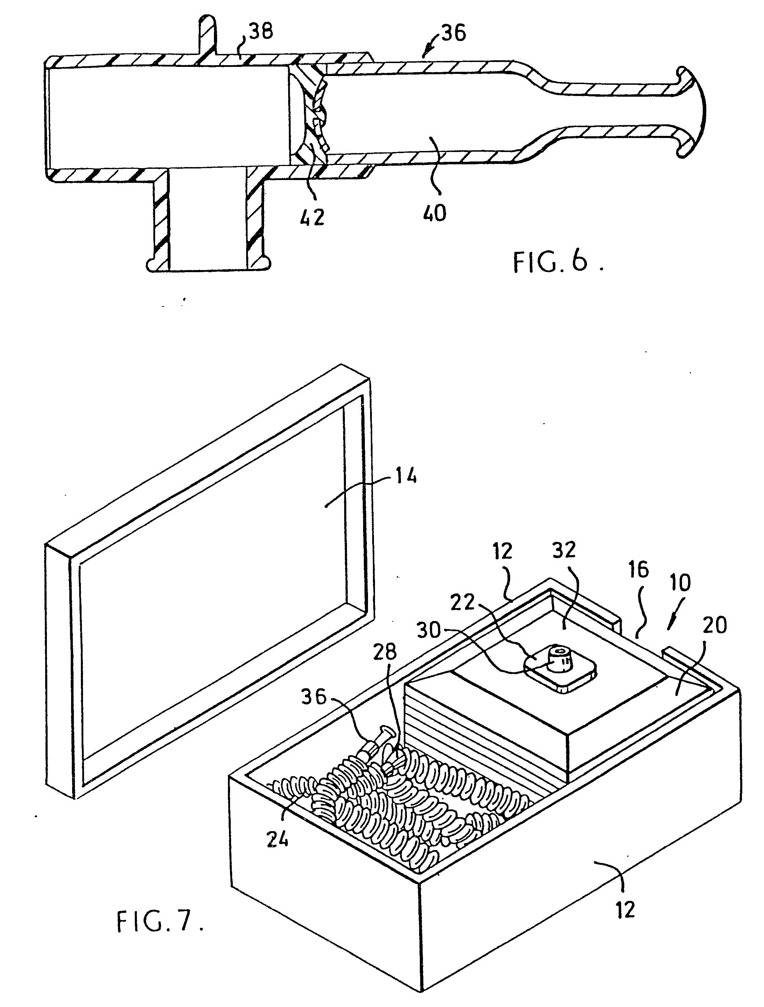

Referring initially with particular reference to Figures 3 to 6 of the accompanying drawings, a volumetric respiratory exerciser 10 includes a bellows enclosure 20 with opposed end plates 32 and 34. The bellows enclosure defines a closed chamber of variable volume and is expandable and retractable between the expanded configuration shown in Figure 5 and the retracted configuration shown in Figure 4. A conduit 24 is in communication at one end with the chamber defined by the bellows enclosure and is connected at its other end with a mouth piece assembly 36. As shown especially in Figure 6, the mouth piece assembly 36 comprises a mouth piece 40 and an air inlet for controlled admission of air.

The exerciser further comprises a support frame 12, 14 for the bellows enclosure 20. As will be appreciated from a study of Figures 4 and 5, the support frame 12, 14 allows movement of the bellows enclosure 20 between its expanded and retracted configurations while retaining in a fixed position the end plate 32 of the bellows enclosure. Indicia 18 are disposed on the frame and serve to indicate the volume of the chamber as it varies with movement relative to the fixed end plate 32 of the bellows enclosure 20.

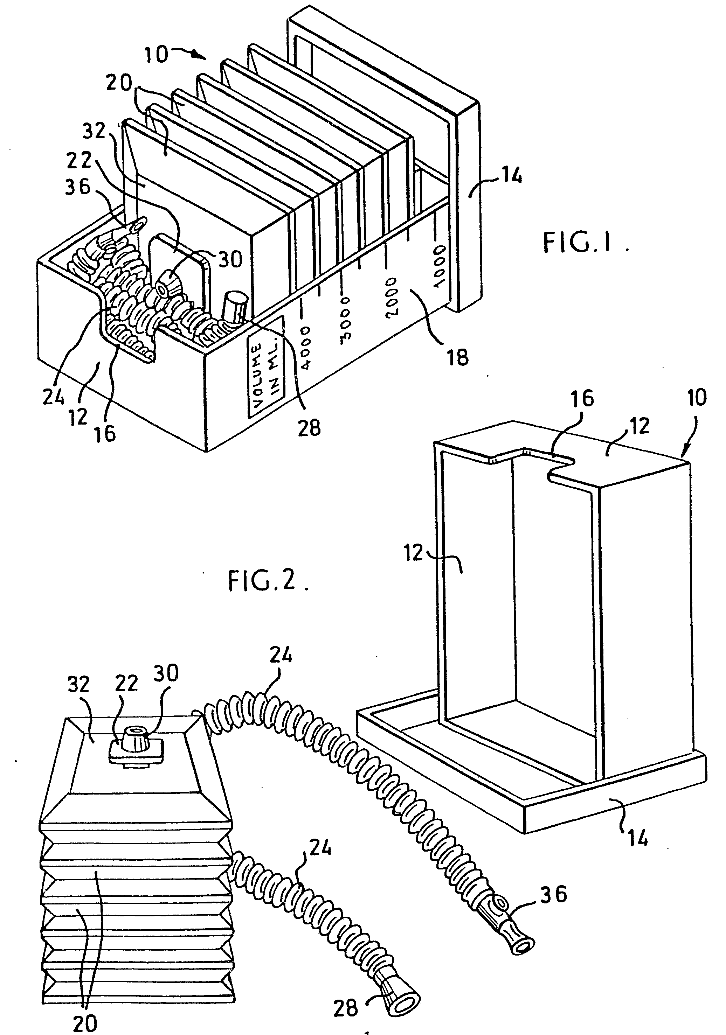

In Figure 1 the bellows enclosure and conduit with mouth piece assembly as shown packaged within a container made from the components, 12, 14 of the support frame. In this instance, the component 12 acts as a carton and the component 14 as a protective end wall. An alternative mode of packaging is shown in Figure 7, where the component 14 then acts as a lid.

The component 12, the carton, has a notch 16 cut in one end and which serves to receive a neck of reduced cross-section between the end plate 32 and an associated flanged section 22.

Figure 2 shows the exerciser 10 in a partly assembled form, with the carton 12 mounted on the support frame components 14 and with its notch 16 at the top. The hose 24 is shown unconnected in Figure 2, and in order to complete the assembly of the exerciser the end 28 of the conduit 24 remote from the mouth piece 36 is secured to a bellows fitting 30 by a frictional sealing fit. The flanged section 22 and end plate 32 are then presented to the notch 16, thereby completing the assembling as shown in Figure 3.

Referring to Figure 3, it will be seen that in its relaxed state the bellows enclosure 20 assumes its expanded configuration. If necessary, additional weights may be fitted to the end plate 34 to ensure that there is expansion. With the bellows enclosure in its expanded configuration it defines a closed chamber with a given maximum volume. This volume can then be varied by withdrawal of air from the chamber.

The indicia 18 on the side of the carton 12 are correlated with a point on the bellows enclosure 20, such as the bottom edge of the bellows enclosure. This correlation is effected in such a manner that the position of the point against the indicia corresponds to the volume reduction of the chamber from its maximum volume. Thus, one can visually ascertain the volume of air removed from the chamber by merely noting the position of the reference point on the bellows enclosure relative to the indicia 18. For example, when the reference point (e.g. the bottom edge of the bellows enclosure) is at the same height as a 3,000 milliliter mark of the indicia 18, this would then mean that an air volume of approximately 3,000 milliliters has been evacuated from the chamber within the bellows enclosure.

Air can be withdrawn from the chamber by use of the conduit 24. This conduit is of flexible hose and is attached to a mouth piece assembly 36. This assembly 30 comprises a moulded mouth piece 40 which is shaped so that it can easily be held by a patient in his mouth. As particularly shown in Figure 6, the assembly 30 additionally comprises a "T"-section conduit 38. The mouth piece 40 is a frictional fit in one opening of the conduit 38. A second opening is attached to the hose 24 while the third opening, the upright limb of the "T" forms an air inlet which can be manually controlled by the patient's thumb. Disposed within the conduit 38 is a one-way valve assembly 42 which allows air to flow from the conduit 24 through the conduit 38 to the mouth piece 40, but not in the opposite direction.

The valve assembly 42 thus serves to ensure that the exerciser 10 is suitable for measuring inspired volumes. Although the valve assembly 42 shown in Figure 6 is of the flexible diaphragm or flap kind, any other suitable design of valve may be employed. Moreover whereas the valve is shown to the mouth piece side of the air inlet opening, it could be arranged to the side of the conduit 24.

The exerciser 10 may be fabricated from any suitable materials and for example the carton 12 and component 14 may be of heavy cardboard, plastics or other rigid or semi-rigid material. The bellows enclosure 20, conduit 24 and mouth piece assembly 36 can be of flexible synthetic polymers such as polyethylene or polypropylene or of other suitable materials.

As mentioned above, the exerciser is shown in an expanded configuration in Figure 5, being the configuration which it normally adopts by virtue of its inherent weight and flexibility. In assuming the position shown in Figure 5, the end plates 32, 34 lie on an axial line through the closed chamber within the bellows enclosure 20. As the bellows enclosure 20 moves towards the retracted configuration shown in Figure 4, the movement is symmetrical with respect to the axial line and the plate 34 remains parallel to the fixed plate 32.

The exerciser 10 is used for measurement of the inspiration volume in the following way:

A patient who is later to be subjected to surgery is first instructed in the use of the exerciser 10. The mouth piece 40 is taken by the patient into his mouth the air inlet of the "T" conduit is covered, and the patient makes a maximal inspiration of air drawing air from the interior chamber of the bellows enclosure 20. Upon so doing, the bellows enclosure 20 moves toward the retracted configuration shown in Figure 4. The position of the reference point on the bellows enclosure 20 with respect to the indicia 18 is noted thereby giving a record of the maximum inspiratory pre-surgical volume of the patient. Since the exerciser 10 is primarily designed so as to be usable by a single patient and then discarded, it is possible for the maximum inspiratory volume to be recorded directly as a mark on the indicia 18. Following measurement of the inspiration volume, the bellows enclosure is allowed to return its its expanded configuration by unclosing of the air inlet of the "T" conduit 38.

If for some reason a pre-surgical maximum inspiratory volume cannot be measured for a particular patient, a calculated expected normal maximum may be assigned to the patient.

Following surgery the patient is asked to make several deep inspirations using the exerciser 10. These deep inspirations are preferably effected at hourly intervals while the patient is awake, with the patient being encouraged to increase gradually the inspired volume until it is back to the pre-surgical level or is higher still. If desired, daily sub-goals for the volume inspirations may be established, either by marking them directly on the indicia 18 or by some other, appropriate means.

When the patient has completed his use of the exerciser 10, the bellows enclosure may be collapsed and the exerciser 10 discarded.

Modifications may be made to the exerciser 10. For example, the end plate 34 may be weighted to increase the power required to move the bellows 20 towards its retracted configuration. In this way the range of usefulness of the exerciser 10 may be increased. Equally the fixed end plate 32 may be secured by various other means apart from use of the flange 22 and notch 16. For example, the end plate 32 may be secured directly with screws, adhesive or the like fastening means to a support mount attached to the carton 12. Furthermore, the indicia may be printed alongside a slot in the side wall of the frame support. The rising and falling of the reference point on the bellows enclosure may then be observed through the slot while the exerciser is being used.

**Claims**

1. A volumetric inspiration respiratory exerciser (10) comprising a bellows enclosure (20) with opposed end plates (32, 34) and defining a closed chamber of variable volume, the bellows enclosure being expandable and retractable between expanded and retracted configurations and the bellows enclosure assuming its expanded configuration when the exerciser is ready for use; a conduit (24) in communication at one end with the chamber and having at its other end a mouth piece assembly (36) comprising an air inlet for controlled admission of air into the chamber and a mouth piece by means of which air is withdrawn from the chamber upon inspiration of the user; a support frame (12, 14) for the bellows enclosure which frame allows movement of the bellows enclosure between its expanded and retracted configurations while retaining in a fixed position one of the end plates of the bellows enclosure

such that the bellows enclosure is suspended from the support frame to assume its expanded configuration; and indicia (18) is disposed on the frame (12) for indicating the volume of the chamber as it varies with movement relative to the fixed end of the bellows enclosure during inspiration by the user.

2. An exerciser according to Claim 1, wherein the bellows enclosure is suspended by suspension means which also serve to fix the position of the said one (32) of the end plates.

3. An exerciser according to claim 2, wherein the bellows enclosure is suspended by engagement between a notch (16) on the support frame and mating member associated with the said one of the end plates.

4. An exerciser according to any preceding claim, wherein during movement of the bellows enclosure (20) towards its retracted configuration, the other end plate (34) of the bellows enclosure moves in a parallel manner towards the fixed end plate (32).

5. An exerciser according to any preceding claim, wherein the mouth piece assembly (36) includes a branched conduit with one opening of the branched conduit forming the air inlet, a second opening being connected to the mouth piece itself, and the third opening being joined to the conduit (24) in communication with the chamber.

6. An exerciser according to any preceding claims, wherein the conduit (24) in communication with the chamber is a flexible hose.

7. An exerciser according to any preceding claim, wherein the support frame (12, 14) is assembled from components used for packaging of the other components.

8. A kit for assembling an exerciser in accordance with claim 1, the kit comprising a container which is adapted for use as the required support frame and which contains the bellows enclosure and conduit with mouth piece assembly.

## Revendications

1. Appareil d'exercice respiratoire d'inspiration volumétrique (10) comprenant une enceinte à soufflet (20) à plaques terminales opposées (32, 34) et définissant une chambre fermée de volume variable, l'enceinte à soufflet étant, expansible et rétractable entre des configurations dilatée et rétractée, et l'enceinte présentant sa configuration dilatée quand l'appareil est prêt à l'emploi; une conduite (24) en communication par une extrémité avec la chambre et portant à son autre extrémité une pièce à embouchure (36) comprenant une entrée d'air pour l'admission contrôlée de l'air dans la chambre et une embouchure proprement dite au moyen de laquelle l'air est évacué de la chambre lorsqu'il y a inspiration par l'utilisateur; un cadre de support (12, 14) pour l'enceinte à soufflet, lequel cadre permet le mouvement de l'enceinte entre ses configurations dilatée et rétractée tout en retenant en position fixe l'une des plaques terminales de l'enceinte de manière que celle-ci soit suspendre au cadre de support pour présenter sa configuration dilatée; et des graduations (18) disposées sur le cadre (12) pour indiquer le volume de la chambre à mesure qu'elle varie quand elle se déplace par rapport à l'extrémité fixe de l'enceinte à soufflet pendant l'inspiration par l'utilisateur.

2. Appareil d'exercice selon la revendication 1, caractérisé en ce que l'enceinte à soufflet est suspendue par des moyens de suspension qui servent également à fixer la position de l'une (32) desdites plaques terminales.

3. Appareil d'exercice selon la revendication 2, caractérisé en ce que l'enceinte à soufflet est suspendue par engagement entre une découpe (16) du cadre de support et un organe correspondant qui est associé à l'une desdites plaques terminales.

4. Appareil d'exercice selon l'une quelconque des revendications précédentes, caractérisé en ce que pendant le mouvement de l'enceinte à soufflet (20) en direction de sa configuration rétractée, l'autre plaque terminale (34) de l'enceinte se déplace en parallèle en direction de la plaque terminale fixe (32).

5. Appareil d'exercice selon l'une quelconque des revendications précédentes, caractérisé en ce que la pièce à embouchure (36) comprend une conduite à dérivation dont une ouverture forme une entrée d'air, une seconde ouverture étant reliée à l'embouchure elle-même et la troisième ouverture étant reliée à la conduite (24) qui est en communication avec la chambre.

6. Appareil d'exercice selon l'une quelconque des revendications précédentes, caractérisé en ce que la conduite (24) qui est en communication avec la chambre est une tuyau flexible.

7. Appareils d'exercice selon l'une quelconque des revendications précédentes, caractérisé en ce que le cadre de support (12, 14) est assemblé à partir des éléments utilisés pour l'emballage des autre éléments.

8. Nécessaire pour l'assemblage d'une appareil d'exercice selon l'une quelconque des revendications précédentes, ce nécessaire comprenant un conteneur qui est adapté à constituer le cadre de support nécessaire, et qui contient l'enceinte à soufflet et la conduite munie de la pièce à embouchure.

## Patentansprüche

1. Volumetrisches Atmungsübungsgerät (10), mit einem Blasebalg (20) mit einander abgekehrten Endplatten (32, 34) und welcher eine abgeschlossene Kammer mit veränderlichem Volumen bildet, wobei der Blasebalg zwischen expandierten und eingezogenen Stellungen ausziehbar und einziehbar ist und seine expandierte Stellung einnimmt, wenn das Übungsgerät betriebsbereit ist; einer Leitung (24), die an einem Ende mit der Kammer in Verbindung steht und an ihrem anderen Ende eine Mundstücksanordnung (36) aufweist, die einen Lufteinlaß für die gesteuerte Zuführung von Luft in die Kammer und ein Mundstück besitzt, durch das Luft aus der Kammer abgesaugt wird beim Einatmen des Benutzers; einem Stützrahment (12, 14) für den Blasebalg, der die Bewegung des Blasebalges zwischen seinen expandierten und seinen eingezogenen Stellungen zuläßt, während er eine der Endplatten des Blasebalges in einer festen Lage zurückhält, derart, daß der Blasebalg am Stützrahmen aufgehängt ist, um seine expandierte Stellung einzunehmen; und am Rahmen (12) angeordneten Markierungen (18) zum Anzeigen des Volumens der Kammer, wie es sich mit der Bewegung relativ zum festen Ende des Blasebalges beim Einatmen des Benützers ändert.

2. Übungsgerät nach Anspruch 1, dadurch gekennzeichnet, daß der Blasebalg mit Hilfe von Aufhängungsmittel aufgehängt ist, die auch zum Fixieren der Lage der genannten einen (32) der Endplatten dienen.

3. Übungsgerät nach Anspruch 2, dadurch gekennzeichnet daß der Blasebalg durch den Eingriff zwischen einer Aussparung (16) am Stützrahmen und einem dazu passenden Gegenstücke, das an der genannten einen der Endplatten angeordnet ist, aufgehängt ist.

4. Übungsgerät nach einem der vorhergehenden Ansprüche, dadurch gekennzeichnet, daß bei der Bewegung des Blasebalges (20) in seine eingezogene Stellung sich die andere Endplatte (34) des Blasebalges parallel gegen die fixierte Endplatte (32) bewegt.

5. Übungsgerät nach einem der vorhergehenden Ansprüche, dadurch gekennzeichnet, daß die Mundstückanordnung (36) einen verzweigten Kanal aufweist, wobei eine Öffnung des verzweigten Kanals den Lufteinlaß bildet, eine zweite Öffnung mit dem Mundstück selbst verbunden ist und die dritte Öffnung mit der mit der Kammer verbundenen Leitung (24) in Verbindung steht.

6. Übungsgerät nach einem der vorhergehenden Ansprüche, dadurch gekennzeichnet, daß die mit der Kammer in Verbindung stehende Leitung (24) eine flexibler Schlauch ist.

7. Übungsgerät nach einem der vorhergehenden Ansprüche, dadurch gekennzeichnet, daß der Stützrahmen (12, 14) aus bestandteilen zusammengebaut, ist, die für die Verpackung der übrigen Bestandteile verwendet werden.

8. Bausatz zum Zusammenbau eines Übungsgerätes nach Anspruch 1, dadurch gekennzeichnet, daß der Bausatz einen Behälter umfaßt, der für die Verwendung als erforderlicher Stützrahmen ausgebildet ist und den Blasebalg und die Leitung mit der Mundstückanordnung enthält.

0 027 154

10

20

32

22

36

30

14

FIG.1.

VOLUME IN ML.

4000

3000

2000

1000

24

12

16

28

18

16

12

10

12

FIG.2.

32

22

30

24

20

24

28

36

14

1

0 0 2 7 1 5 4

FIG. 3.

FIG. 4

FIG. 5.

2

FIG. 6 .

FIG. 7 .